(19) **Europäisches Patentamt**
**European Patent Office**
**Office européen des brevets**

(11) Veröffentlichungsnummer : **0 384 312 B1**

(12) # EUROPÄISCHE PATENTSCHRIFT

(45) Veröffentlichungstag der Patentschrift :
**30.10.91 Patentblatt 91/44**

(51) Int. Cl.$^5$ : **B01J 23/66, C07D 301/10, B01J 35/10, B01J 37/02**

(21) Anmeldenummer : **90103018.9**

(22) Anmeldetag : **16.02.90**

(54) Verfahren zur Herstellung eines Silberkatalysators.

(30) Priorität : 23.02.89 DE 3905578

(43) Veröffentlichungstag der Anmeldung :
**29.08.90 Patentblatt 90/35**

(45) Bekanntmachung des Hinweises auf die
Patenterteilung :
**30.10.91 Patentblatt 91/44**

(84) Benannte Vertragsstaaten :
**BE DE ES FR GB IT NL SE**

(56) Entgegenhaltungen :
**EP-A- 0 099 975
EP-A- 0 168 782
EP-A- 0 211 397
DE-A- 2 819 595**

(72) Erfinder : **Herzog, Klaus, Dr.
Oggersheimer Strasse 112
W-6700 Ludwigshafen (DE)**
Erfinder : **Boehning, Karl-Heinz, Dr.
Mecklenburger Strasse 14
W-6100 Darmstadt (DE)**
Erfinder : **Aichinger, Heinrich, Dr.
Rathenaustrasse 6
W-6800 Mannheim 1 (DE)**
Erfinder : **Plueckhan, Juergen, Dr.
Bensheimer Ring 19 b
W-6710 Frankenthal (DE)**
Erfinder : **Mross, Wolf Dieter, Dr.
Anselm-Feuerbach-Strasse 21
W-6710 Frankenthal (DE)**
Erfinder : **Schwarzmann, Matthias, Dr.
Carl-Bosch-Strasse 54
W-6703 Limburgerhof (DE)**

(73) Patentinhaber : **BASF Aktiengesellschaft
Carl-Bosch-Strasse 38
W-6700 Ludwigshafen (DE)**

## Beschreibung

Die vorliegende Erfindung betrifft ein Verfahren zur Herstellung von Silber und Alkalimetalle enthaltenden Trägerkatalysatoren in einem Zweistufenprozeß, wobei ein Silber in metallischer Form und gegebenenfalls Lithium enthaltender Grundkatalysator mit den schweren Alkalimetallen Natrium, Kalium, Rubidium und/oder Cäsium dotiert wird.

Des weiteren betrifft die Erfindung ein Verfahren zur Herstellung von Ethylenoxid.

Katalysatoren zur Herstellung von Ethylenoxid, welche neben metallischem Silber die schweren Alkalimetalle Natrium, Kalium, Rubidium und/oder Cäsium und gegebenenfalls zusätzlich das leichte Alkalimetall Lithium enthalten, sind beispielsweise aus DE-A-2300512 oder aus DE-A-2819595 bekannt. Obwohl die Herstellung dieser Katalysatoren technisch als relativ ausgereift gelten kann, besteht aufgrund des Umstandes, daß Ethylenoxid eine in großen Mengen produzierte Grundchemikalie ist, ein steter Bedarf nach verbesserten Herstellungsmethoden, die es erlauben, die Eigenschaften dieser Katalysatoren wie Selektivität, Aktivität und Langzeitstabilität noch weiter zu verbessern. Dementsprechend ist die Entwicklung verbesserter Herstellmethoden für derartige Silberkatalysatoren ein stark beforschtes Gebiet, wobei sich ein Großteil der Arbeiten mit der optimalen Aufbringung der einzelnen Katalysatorkomponenten auf den Träger beschäftigt.

Über die optimale Art wie das Silber und die Alkalimetalle auf den Träger — im allgemeinen wird als Träger α-Aluminiumoxid verwendet — aufzubringen sind, ob zusammen oder getrennt und wenn getrennt, in welcher Reihenfolge oder über die Verwendung von Hilfsmitteln zur Imprägnierung, wie Komplexbildnern, Tensiden, Reduktionsmitteln usw. (vgl. EP-B 14457) gibt es keine allgemein anerkannte Lehre und die Ansichten zu diesen Punkten differieren in starkem Maße.

So wird in DE-A 2300512 die gleichzeitige, "einstufige" Aufbringung des Silbers und der Alkalipromotoren als besonders vorteilhaft hervorgehoben. Aus EP-A 168782 ist bekannt, daß die Wirkung von Silberkatalysatoren zur Ethylenoxidherstellung, deren Selektivität mit fortschreitender Betriebsdauer abgenommen hat, verbessert werden kann, wenn man sie mit einem schweren Alkalimetallkation wie Rubidium und insbesondere Cäsium dotiert.

Des weiteren lehrt diese Patentschrift, daß "einstufig" oder "zweistufig" (1. Stufe : Aufbringung von Silber; 2. Stufe : Aufbringung der Alkalimetallpromotoren) hergestellte Katalysatoren einander dann gleichwertig sind, wenn die nachträgliche Aufbringung der schweren Alkalimetallpromotoren an einem thermisch stabilisierten, ungebrauchten Silberkatalysator vorgenommen wird. Indessen kann nach DE-A 2819595 ein "zweistufig" hergestellter Katalysator (1. Stufe : Aufbringung von Silber und Lithium ; 2. Stufe : Aufbringung der schweren Alkalimetallpromotoren) erhalten werden, der bei gleicher Selektivität aktiver ist als ein "einstufig" hergestellter Katalysator, wenn man die schweren Alkalimetallpromotoren in der 2. Stufe in Gegenwart einer oberflächenaktiven Substanz auf einen Silber und Lithium enthaltenden Grundkatalysator aufbringt.

Der Erfindung lag nun die Aufgabe zugrunde, ein verbessertes Herstellungsverfahren, insbesondere ein verbessertes Dotierungsverfahren, zur Herstellung von schwere Alkalimetallpromotoren enthaltenden Silberkatalysatoren zu finden, das es erlaubt, leistungsfähigere Katalysatoren zur Ethylenoxidherstellung zu produzieren.

Dementsprechend wurde ein Verfahren zur Herstellung von Silber und Alkalimetalle enthaltenden Trägerkatalysatoren in einem Zweistufenprozeß, wobei ein Silber in metallischer Form und gegebenenfalls Lithium enthaltender Grundkatalysator mit den schweren Alkalimetallen Natrium, Kalium, Rubidium und/oder Cäsium dotiert wird, gefunden, das dadurch gekennzeichnet ist, daß man die Dotierung mit einer die schweren Alkalimetalle enthaltenden Imprägnierlösung ausführt, welche ein Nitrat oder eine Mischung von Nitraten der allgemeinen Formel

$$R^+NO_3^-$$

enthält, worin $R^+$ für ein Ammonium-, Hydrazinium-, $C_1$- bis $C_{12}$-Monoalkylammonium-, $C_2$- bis $C_{12}$-Dialkylammonium-, $C_3$- bis $C_{12}$-Trialkylammonium- oder ein $C_4$- bis $C_{12}$-Tetraalkylammonium-Kation steht.

Des weiteren wurde ein Verfahren zur Herstellung von Ethylenoxid gefunden, das dadurch gekennzeichnet ist, daß man einen Katalysator verwendet, der wie oben angegeben hergestellt worden ist.

Das erfindungsgemäße Verfahren betrifft somit praktisch nur Stufe 2 des Zweistufenverfahrens zur Herstellung der mit schweren Alkalimetallkationen dotierten Silber-Trägerkatalysatoren.

Die Maßnahmen der Stufe 1, also die Aufbringung des Silbers und gegebenenfalls des Lithiums auf den Träger und die anschließende Wärmebehandlung, mithin die Herstellung des Grundkatalysators, sind nicht Gegenstand der vorliegenden Erfindung. Zur Herstellung des Grundkatalysators wird deshalb auf den Stand der Technik, beispielsweise auf DE-A 2819595, EP-A 168782 und auf EP-A 271814 verwiesen.

Erfindungsgemäß werden der Imprägnierlösung, welche die als Promotoren dienenden, schweren Alkalimetallkationen enthält, Nitrate der Formel

$$R^+NO_3^-$$

zugesetzt. Bei dem Rest R+ handelt es sich um Ammonium-, Hydrazinium- oder Alkylammonium-, wie $C_1$- bis $C_{12}$-Monoalkylammonium-, $C_2$- bis $C_{12}$-Dialkylammonium-, $C_3$- bis $C_{12}$-Trialkylammonium oder $C_4$- bis $C_{12}$-Tetraalkylammonium-Kationen, wobei solche Ammonium-Kationen $R^+$, die nicht mehr als 8 und insbesondere nicht mehr als 4 Kohlenstoffatome in ihrem Alkylteil enthalten, bevorzugt werden. Besonders wirksam und daher besonders bevorzugt sind unsubstituierte Ammonium- und Hydraziniumnitrate sowie Hydraziniumdinitrate.

Unter dem Begriff Alkylammonium-Kationen sind selbstverständlich auch Ammonium-Kationen von Alkylendiaminen, Triaminen und Tetraaminen zu verstehen, die in ihrer Wirkung den genannten Alkylammonium-Kationen äquivalent sind. Die Art der Alkylgruppen in den Alkylammonium-Kationen ist nicht kritisch, d.h. sie können sowohl unverzweigte oder verzweigte Alkylgruppen, als auch Cycloalkylgruppen enthalten. Selbstverständlich können auch Gemische verschiedener Nitrate $R^+NO_3^-$ beim erfindungsgemäßen Verfahren benutzt werden, ohne daß dies seinem Erfolg abträglich wäre.

Die betreffenden Ammonium-, Hydrazinium- und Alkylammonium-Nitrate, im folgenden kurzerhand als "Nitrate" bezeichnet, werden in einer Imprägnierlösung auf den Silber und gegebenenfalls Lithium enthaltenden Grundkatalysator aufgebracht. Diese Imprägnierlösung enthält außer den Nitraten und einem wasserlöslichen organischen Lösungsmittel, Wasser und den oder die aufzubringenden Alkalimetallpromotoren.

Als wasserlösliche organische Lösungsmittel eignen sich insbesondere wasserlösliche Amine wie sec.-Butylamin, tert.-Butylamin, Propylamin und Isopropylamin, aber auch mit Alkoholen, wie Methanol, Ethanol und Isopropanol werden gute Ergebnisse erzielt. Auch andere polare, wasserlösliche organische Lösungsmittel, beispielsweise Formamide und Alkylformamide sind geeignet.

Der Alkalimetallpromotor wird zweckmäßigerweise gelöst in Wasser zur Imprägnierlösung gegeben, deren Wassergehalt eine Ausfällung des Alkalimetallsalzes verhindert. Das Anion des Alkalimetallsalzes kann im Prinzip frei gewählt werden ; als besonders vorteilhaft empfiehlt sich jedoch die Anwendung der Alkalimetallnitrate.

Die genaue Wirkungsweise der "Nitrate" bei der Imprägnierung des Katalysators mit Alkalimetallkationen ist noch nicht aufgeklärt, doch ist offenbar ihre vorteilhafte Wirkung auf die Eigenschaften der so hergestellten Silberkatalysatoren eher auf das Nitrat-Anion als auf das Ammonium-Kation zurückzuführen. Im Einklang hiermit ist die Beobachtung, daß mit steigender Zahl der Kohlenstoffatome im Ammonium-Kation, d.h. also mit der Zunahme des Ammoniumkation-Anteils am Molekulargewicht des Ammoniumnitrates, die Wirksamkeit der Ammoniumnitrate entsprechend abnimmt und durch die Erhöhung der zugesetzten Menge an Alkylammoniumnitrat kompensiert werden muß.

Im allgemeinen werden, bezogen auf den Grundkatalysator, 1 bis 30, vorzugsweise 3 bis 25 und besonders vorteilhaft 5 bis 15 Gew.-% Ammoniumnitrat ($NH_4NO_3$) auf den Grundkatalysator aufgebracht. Alkylammoniumnitrate werden im allgemeinen entsprechend ihrem im Vergleich zu Ammoniumnitrat geringeren Nitratgehalt höher dosiert. Da sich die Wirksamkeit der Nitrate $R^+NO_3^-$ und der Nitratgehalt im jeweiligen $R^+NO_3^-$-Molekül nicht direkt proportional zueinander verhalten, empfiehlt es sich, bei Verwendung von Alkylammoniumnitraten die jeweils optimale Menge in einem Laborversuch zu ermitteln.

Zweckmäßigerweise wird die Imprägnierlösung in relativ konzentrierter Form angewandt, mit der Maßgabe, daß sie so konzentriert ist, daß einesteils Nitrate und Alkalimetallpromotoren gelöst bleiben und nicht ausfallen und andernteils das Volumen dieser Lösung so gering bleibt, daß sie beim Vermengen mit einer bestimmten Menge des zu imprägnierenden Grundkatalysators von dieser vollständig aufgesogen wird. Das Gesamtvolumen und die Zusammensetzung der Imprägnierlösung aus den Komponenten organisches Lösungsmittel, Wasser, Nitrat und Alkalimetallpromotor bzw. Alkalimetallpromotorlösung kann innerhalb dieses weiten Rahmens variieren. Die innerhalb dieses Rahmens jeweils eben noch mögliche Höchst- und Mindestmenge einer dieser Einzelkomponenten in diesem 4-Komponenten-Gemisch hängt von der Art und Menge der anderen mitverwendeten Komponenten und selbstverständlich auch von den Sorptionseigenschaften des Grundkatalysators ab, so daß keine generelle Regel gegeben werden kann, nach welcher die Konzentrationen der jeweils verwendeten Einzelkomponenten zueinander in Beziehung stehen sollen. Im allgemeinen lassen sich geeignete Mischungsverhältnisse leicht durch einige wenige Mischungsversuche im Reagenzglas ermitteln.

Im allgemeinen werden die Alkalimetallpromotoren mit Hilfe einer derartigen Imprägnierlösung so auf den Grundkatalysator aufgebracht, daß dieser, wie oben erwähnt, die Imprägnierlösung vollständig adsorbiert. Dabei kann es sich als vorteilhaft erweisen, wenn das so erhaltene Gemenge sich noch einige Zeit zum Zwecke der Äquilibrierung selbst überlassen bleibt. Zweckmäßig ist dazu eine Äquilibrierzeit von 0,5 bis 6 Stunden.

Anschließend wird der Katalysator getempert und zwar zweckmäßigerweise in einem Stickstoffstrom bei Temperaturen zwischen 180 und 300°C, vorteilhaft zwischen 200 und 250°C und insbesondere zwischen 220 und 250°C. Bei diesen Temperaturen zersetzt sich das zugesetzte Nitrat in der Regel in wenigen Minuten und entfaltet dabei möglicherweise seine Wirkung. Der so erhaltene Katalysator kann direkt zur Ethylenoxidherstellung verwendet werden.

Katalysatoren mit besonders günstigen Eigenschaften werden nach dem vorliegenden Dotierungsverfahren erhalten, wenn die schweren Alkalimetalle Natrium, Kalium, Rubidium und/oder Cäsium in Mengen von, bezogen auf den Gesamtkatalysator, 0,0005 bis 0,06 Gew.-% auf dem Grundkatalysator abgeschieden werden.

Als besonders vorteilhaft wirkt sich das vorliegende Imprägnierverfahren auf die Leistungsfähigkeit solcher Silberkatalysatoren aus, welche Silbergehalte zwischen 2 und 18 Gew.-% haben und Lithium in Mengen zwischen 0,0005 und 0,3 Gew.-%, bezogen auf den Gesamtkatalysator, enthalten.

Als Trägermaterial eignet sich für das vorliegende Verfahren zur Herstellung von Silberkatalysatoren $\alpha$-Aluminiumoxid, insbesondere $\alpha$-Aluminiumoxid einer Reinheit von mehr als 99% und einer BET-Oberfläche von 0,4 m²/g bis 0,8 m²/g und mit einem Porenvolumen von mindestens 0,4 cm³/g.

Die Herstellung von Ethylenoxid kann mit den erfindungsgemäß hergestellten Silberkatalysatoren auf die übliche Art und Weise erfolgen, wie sie beispielsweise aus den zum Stand der Technik referierten Schriften entnommen werden kann. Die nach dem erfindungsgemäßen Verfahren erhältlichen Silberkatalysatoren zeichnen sich überraschenderweise durch eine hohe Aktivität, eine hohe Selektivität und eine hohe Langzeitstabilität aus.

Beispiele

Bei den beschriebenen Versuchen wurde als Trägermaterial ein $\alpha$-Aluminiumoxid verwendet, das den in der Tabelle 1 aufgeführten Anforderungen entsprach.

Tabelle 1: Anforderungen an das Trägermaterial

| | |
|---|---|
| Gehalt an $\alpha$-Al$_2$O$_3$ | ca. 99 Gew.-% |
| Gehalt an SiO$_2$ | ca. 0,1 bis 0,5 Gew.-% |
| lösliche Ionen | |
| Al | 250 bis 2000 Gew.-ppm |
| Ca | 150 bis 2000 Gew.-ppm |
| K | 20 bis 1000 Gew.-ppm |
| Na | 50 bis 1000 Gew.-ppm |
| BET-Oberfläche | 0,4 bis 0,8 m²/g |
| Wasseraufnahme | |
| kalt (20°C) | 0,4 bis 0,5 cm³/g |
| heiß (100°C) | 0,45 bis 0,55 cm³/g |
| Schüttgewicht | 600 bis 800 kg/m³ |

Herstellung des Grundkatalysators :

Der Grundkatalysator wurde nach dem Verfahren der EP-A 271814 hergestellt :
100 g des $\alpha$-Aluminiumoxid-Trägers wurden mit einer Lösung aus 24,2 g sec.-Butylamin, 5,9 g Wasser, 27,3 g Silbernitrat und 160 mg Lithiumnitrat imprägniert, 3 Stunden bei Raumtemperatur gelagert und anschließend in einen Umluftofen überführt und bei 240°C im Stickstoffstrom getempert. Der so erhaltene Grundkatalysator enthielt 14 Gew.-% Silber und 130 Gew.-ppm Lithium.

Beispiel 1

100 g Grundkatalysator wurden in einem Mischer mit einer Lösung versetzt, welche 17,4 g sec.-Butylamin, 4,7 g Wasser, 13,9 g Ammoniumnitrat und 0,48 ml einer Cäsiumnitratlösung enthielt, deren Cäsiumgehalt 68,2 g Cäsium/l betrug. Nachdem die Imprägnierflüssigkeit vollständig vom Grundkatalysator aufgesogen worden war, wurde das so erhaltene Katalysatorvorprodukt 3 h bei Raumtemperatur gelagert und anschließend im Umluftofen bei 230°C unter einem Stickstoffstrom 20 Minuten lang getempert. Der so hergestellte Katalysator (im folgenden mit A bezeichnet) enthielt neben den Bestandteilen des Grundkatalysators 300 Gew.-ppm Cäsium.

Beispiel 2

Der Katalysator wurde wie in Beispiel 1 beschrieben hergestellt, die Imprägnierlösung enthielt jedoch nur 10,6 g Ammoniumnitrat (im folgenden wird dieser Katalysator als B bezeichnet).

Beispiel 3 (Vergleichsbeispiel)

Der Katalysator wurde wie in Beispiel 1 beschrieben hergestellt. Die Imprägnierlösung enthielt jedoch kein Ammoniumnitrat (im folgenden wird dieser Katalysator als C bezeichnet).

Herstellung von Ethylenoxid

Die in den Beispielen 1 bis 3 hergestellten Katalysatoren wurden zerkleinert und gesiebt. Jeweils 10 g der Siebfraktion von 0,6 bis 0,75 mm wurden in einen thermostatisierbaren Reaktor aus rostfreiem Stahl von 5 mm Innendurchmesser gefüllt. Durch den Reaktor wurde ein Gasgemisch der Zusammensetzung 30% Ethylen, 8% Sauerstoff, 4% Kohlendioxid und 5 ppm Vinylchlorid (Inhibitor), Rest Stickstoff, geleitet. Der Druck wurde auf 16 bar eingestellt. Die Katalysatorbelastung betrug 3300 Nl Gas/l Kontakt. Die Temperatur wurde so geregelt, daß der Sauerstoffumsatz 30% betrug. Nach 3 Tagen Laufzeit wurden Proben gezogen und die Aktivität und Selektivität des Katalysators bestimmt. Die ermittelten Daten sind der Tabelle 2 zu entnehmen.

Tabelle 2

| Katalysator | Aktivität T (°C) | Selektivität S (%) |
|---|---|---|
| A | 220 | 82,5 |
| B | 217 | 82,8 |
| C | 216 | 81,7 |

## Patentansprüche

1. Verfahren zur Herstellung von Silber und Alkalimetalle enthaltenden Trägerkatalysatoren in einem Zweistufenprozeß, wobei ein Silber in metallischer Form und gegebenenfalls Lithium enthaltender Grundkatalysator mit den schweren Alkalimetallen Natrium, Kalium, Rubidium und/oder Cäsium dotiert wird, dadurch gekennzeichnet, daß man die Dotierung mit einer die schweren Alkalimetalle enthaltenden Imprägnierlösung ausführt, welche ein Nitrat oder eine Mischung von Nitraten der allgemeinen Formel

$$R^+NO_3^-$$

enthält, worin $R^+$ für ein Ammonium-, Hydrazinium-, $C_1$- bis $C_{12}$-Monoalkylammonium-, $C_2$- bis $C_{12}$-Dialkylammonium-, $C_3$- bis $C_{12}$-Trialkylammonium oder ein $C_4$- bis $C_{12}$-Tetraalkylammonium-Kation steht.

2. Verfahren nach Anspruch 1, dadurch gekennzeichnet, daß man einen Grundkatalysator verwendet, der 2 bis 18 Gew.-% Silber und 0,0005 bis 0,3 Gew.-% Lithium enthält.

3. Verfahren nach Anspruch 1, dadurch gekennzeichnet, daß man mit der Imprägnierlösung 0,0005 bis 0,06 Gew.-% Natrium, Kalium, Rubidium und/oder Cäsium auf dem Träger abscheidet.

4. Verfahren nach Anspruch 1, dadurch gekennzeichnet, daß man ein Trägermaterial verwendet, das zu mehr als 99 Gew.-% aus hochreinem $\alpha$-Aluminiumoxid besteht und eine BET-Oberfläche von 0,4 m²/g bis 0,8 m²/g hat und dessen Porenvolumen mindestens 0,4 cm³/g beträgt.

5. Verfahren gemäß Anspruch 1, dadurch gekennzeichnet, daß die Verbindung $R^+NO_3^-$ in Mengen von 1 bis 30 Gew.-% bezogen auf den Grundkatalysator, angewendet wird.

6. Verfahren zur Herstellung von Ethylenoxid aus Ethylen und Sauerstoff in der Gasphase, mittels eines silberhaltigen Katalysators, dadurch gekennzeichnet, daß man einen Katalysator verwendet, der gemäß den Ansprüchen 1 bis 5 hergestellt worden ist.

## Claims

1. A two-stage process for preparing a supported catalyst which contains silver and an alkali metal by doping a basic catalyst which contains silver in metallic form and may also contain lithium with the heavy alkali metal sodium, potassium, rubidium or cesium using an impregnating solution which contains the heavy alkali metal and one or more nitrates of the general formula

$$R^+NO_3^-$$

where $R^+$ is an ammonium, hydrazinium, $C_1$-$C_{12}$-monoalkylammonium, $C_2$-$C_{12}$-dialkylammonium, $C_3$-$C_{12}$-trialkylammonium or $C_4$-$C_{12}$-tetraalkylammonium cation.

2. A process as claimed in claim 1, wherein the basic catalyst used contains 2-18% by weight of silver and 0.0005-0.3% by weight of lithium.

3. A process as claimed in claim 1, wherein the impregnating solution deposits from 0.0005 to 0.06% by weight of sodium, potassium, rubidium or cesium on the carrier.

4. A process as claimed in claim 1, wherein the carrier material used is more than 99% by weight high-purity α-alumina and has a BET surface area of 0.4-0.8 m²/g and a pore volume of not less than 0.4 cm³/g.

5. A process as claimed in claim 1, wherein the compound $R^+NO_3^-$ is employed in an amount of from 1 to 30% by weight, based on the basic catalyst.

6. A process for preparing ethylene oxide from ethylene and oxygen in the gas phase by means of a silver-containing catalyst, which comprises using a catalyst prepared as claimed in claim 1 or 2 or 3 or 4 or 5.

## Revendications

1. Procédé de préparation de catalyseurs fixés sur support contenant de l'argent et des métaux alcalins, en une opération en deux temps dans laquelle un catalyseur de base, contenant de l'argent sous forme métallique et éventuellement du lithium, est dopé avec les métaux alcalins lourds sodium, potassium, rubidium et/ou césium, caractérisé en ce qu'on procède au dopage avec une solution d'imprégnation qui renferme les métaux alcalins lourds et qui contient un nitrate ou un mélange de nitrates de formule générale

$$R^+NO_3^-$$

dans laquelle $R^+$ est mis pour un ion ammonium, hydrazinium, $C_1$-$C_{12}$-monoalkylammonium, $C_2$-$C_{12}$-dialkylammonium, $C_3$-$C_{12}$-trialkylammonium ou pour un cation $C_4$-$C_{12}$-tétraalkylammonium.

2. Procédé selon la revendication 1, caractérisé en ce qu'on utilise un catalyseur de base qui contient de 2 à 18% en poids d'argent et de 0,0005 à 0,3% en poids de lithium.

3. Procédé selon la revendication 1, caractérisé en ce qu'on dépose sur le support, au moyen de la solution d'imprégnation, de 0,0005 à 0,06% en poids de sodium, de potassium, de rubidium et/ou de césium.

4. Procédé selon la revendication 1, caractérisé en ce qu'on utilise une matière de support qui se compose, pour plus de 99% en poids, d'oxyde d'aluminium α de pureté élevée et qui a une une surface spécifique BET de 0,4 m²/g à 0,8 m²/g et un volume de poids d'au moins 0,4 cm³/ g.

5. Procédé selon la revendication 1, caractérisé en ce que le composé $R^+NO_3^-$ est utilisé dans des proportions de 1 à 30% en poids par rapport au catalyseur de base.

6. Procédé de préparation d'oxyde d'éthylène à partir d'éthylène et d'oxygène en phase gazeuse, au moyen d'un catalyseur contenant de l'argent, caractérisé en ce qu'on utilise un catalyseur qui a été préparé selon l'une quelconque des revendications 1 à 5.